# EUROPEAN PATENT SPECIFICATION

(11) **EP 3 071 247 B1**
(45) Date of publication and mention of the grant of the patent: **17.04.2019**
(21) Application number: 14812632.9
(22) Date of filing: 17.11.2014
(51) Int. Cl.: A61L 27/18, A61L 27/56, A61L 27/60

(54) **DEVICE FOR THE CONSTRUCTION OF SKIN**
VORRICHTUNG ZUR HAUTKONSTRUKTION
DISPOSITIF POUR LA CONSTRUCTION DE LA PEAU

(30) Priority: 18.11.2013 IT MI20131904
(43) Date of publication of application: 28.09.2016
(73) Proprietor: Sambusseti, Antonio, 26100 Cremona (IT)
(72) Inventor: Sambusseti, Antonio, 26100 Cremona (IT)
(74) Representative: Ripamonti, Enrico
(86) International application number: PCT/IB2014/066092
(87) International publication number: WO 2015/071880

(56) References cited:
- EP-A1- 0 462 426
- EP-A1- 1 216 718
- WO-A2-2009/093023

## Description

The subject of the present invention is a device for skin reconstruction.

The present invention can be advantageously applied in the skin reconstruction operations of patients who have suffered serious burns.

In these situations, it is necessary to reconstruct the destroyed skin in a manner so as to restore the compromised functions thereof, foremost of these being that of protective barrier.

Autograft is one of the techniques employed; it provides for drawing a skin portion from one zone of the patient body that was not compromised.

Such skin portion is subsequently treated before being applied. In particular, it is cut in a manner so as to achieve a plurality of holes.

The portion thus perforated allows, on one hand, covering the burnt zone and temporarily protecting it, while on the other hand it allows a suitable oxygenation of the burnt zone in a manner so as to facilitate the natural reconstruction of the skin.

The skin reconstruction occurs starting from the edges of the holes towards their center. During such step, the patient is maintained in a controlled environment and atmosphere - the so-called clean rooms - in order to prevent infections.

However, this technique has several considerable drawbacks.

Clearly, the drawing of the non-damaged skin portion consists of a further surgical operation on the burnt patient, which causes a further scar, even if in a zone of the body that is not visible.

In addition, such technique can only be used if the skin of the patient is not completely compromised, so that a skin portion on which the drawing can be performed is identifiable.

Other known techniques for repairing and/or regenerating skin are disclosed in WO2009/093023 which provides a bioresorbable synthetic scaffold suitable for supporting the migration and proliferation of human soft tissue cells and comprising at least two fibrous materials and wherein at least the first fibrous material comprises pores. Another known solution is disclosed in EP0462426 which relates to a biocompatible perforate membrane which is used as a support in the in vitro growth of epithelial cells to be used for obtaining artificial skin.

In this context, the technical task underlying the present invention is to propose a device for skin reconstruction that overcomes the abovementioned drawbacks of the prior art.

In particular, object of the present invention is to provide a device for skin reconstruction which allows a quick reconstruction of the skin and which is applicable in any situation.

The specified technical task and object are substantially attained by a device for skin reconstruction comprising the technical characteristics set forth below in one or more of the enclosed claims.

Further characteristics and advantages of the present invention will be clearer from the exemplifying and hence non-limiting description of a preferred but not exclusive embodiment of a device for skin reconstruction, as illustrated in the enclosed drawings in which:
- figure 1 is a schematic perspective view of a device for skin reconstruction in accordance with the present invention;
- figure 2 is a plan view of an embodiment variant of the device of figure 1; and
- figure 3 is a section view of an embodiment variant of the device of figure 2 along a section line III-III.

With reference to the enclosed figures, reference number 1 overall indicates a device for the reconstruction of the skin of patient who has suffered a burn in accordance with the present invention.

The device 1 comprises a piece of fabric 2. The piece 2 is intended to be placed above a portion of the patient body that has been partly or completely damaged following a burn.

In particular, the fabric is made of PGA (polyglycolide or poylglycolic acid), preferably homopolymer. Still more in particular, the fabric is made with an ultralight monofilament or thread deriving from PGA fibers.

PGA is a highly biocompatible and resorbable polymer. In detail, the resorption time of PGA is approximately one month.

The fabric of the piece 2 can be obtained by weaving the PGA thread in various ways, giving rise to a knitted fabric, a woven fabric or a non-woven fabric.

Preferably, the fabric is a knitted fabric, still more preferably a warp knitted fabric.

In this case, the fabric has a rougher surface capable of assuming a net configuration with sufficiently small meshes.

In detail, its weft is such that its interstitial space is less than 200 pm, preferably around 160 pm, corresponding to an average area of the holes equal to approximately 0.02 mm². This ensures good protection of the fabrics covered by the piece 2.

Furthermore, the fabric is preferably textured so as to give it even greater surface roughness and greater rigidity and impermeability.

In addition, the fabric is preferably obtained with a thread having a density comprised between 50 and 200 denier.

Merely by way of example, the fabric has a thickness substantially comprised between 0.3 mm and 0.6 mm, more preferably comprised between 0.4 mm and 0.53 mm, and still more preferably is substantially 0.45 mm.

The piece 2 has a plurality of holes 3 arranged over the entire surface of the piece 2 itself.

The piece 2 thus perforated allows a protection of the portion damaged by the burn and, simultaneously, a suitable oxygenation of the tissues in order to allow the natural reconstruction of the skin.

In accordance with that illustrated, the holes 3 are all equivalent to each other, both with regard to shape and size.

In detail, the holes 3 are aligned along straight and preferably orthogonal lines. In other words, the holes 3 are arranged in a manner so as to form a regular lattice. In the illustrated and preferred embodiment, the holes 3 have rhomboid shape. More in detail, the sides of each hole 3 are equivalent and orthogonal to each other. In other words, in the preferred embodiment the holes 3 have square form, but are arranged in a manner such that the vertices of the adjacent holes 3 are adjacent to each other.

In an alternative embodiment, the holes 3 have square form, but are arranged in a manner such that the sides of the adjacent holes 3 are adjacent to each other.

In further embodiments, the holes can be rectangular, circular or any other shape.

In the preferred embodiment, the holes 3 have sides with length comprised between 0.5 cm and 1 cm.

The distance between the adjacent holes 3 is comprised between 0.5 cm and 1 cm.

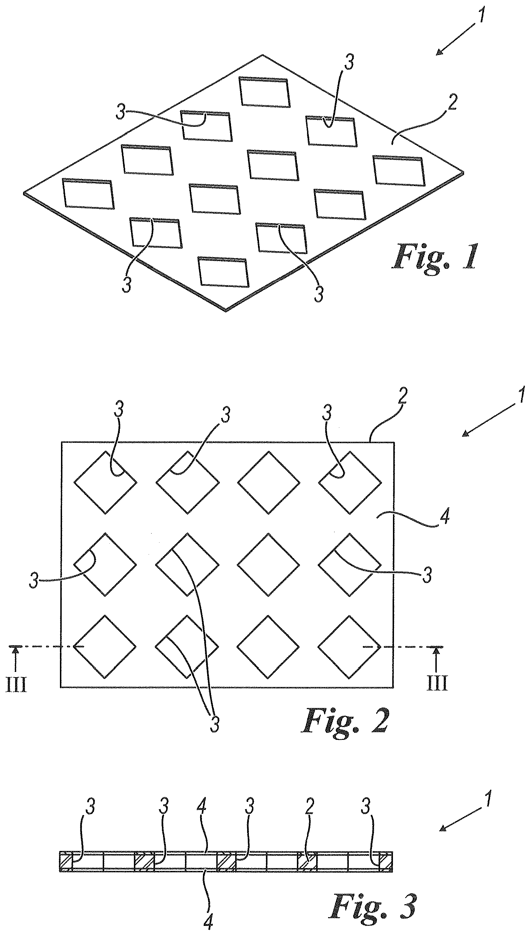

In the invention and as illustrated in figures 2 and 3, the device 1 further comprises at least one coating layer 4 applied on at least one of the surfaces of said piece 2, wherein the coating layer 4 is made of collagen, preferably purified collagen of porcine origin. Preferably, the device 1 comprises two coating layers 4, each applied to a respective surface of the piece 2. Advantageously, the use of the coating layers 4 with collagen allows speeding up and improving the skin reconstruction process.

The size of the piece 2 can be totally variable and depends on the extension of the skin zone destroyed by the burn.

During use, the device 1 is as said used by applying it to the zones of the patient that have been damaged after a burn.

The device 1 simultaneously provides protection for the damaged zone and correct transpiration, necessary for facilitating the natural reconstruction of the skin. During the reconstruction phase, the piece 2 is decomposed, being dissolved over a period of about one month.

Alternatively, the device 1 can be used in combination with the known autograft techniques, if possible.

In other words, this method comprises the step of drawing a healthy skin portion from the patient.

Optionally, such skin portion can be subjected, or not subjected, to the known phase of perforation.

The device 1 is then applied to the damaged zone of the patient and the skin portion drawn from the patient is applied above the device 1.

Also in this case, the device 1 is dissolved while the regeneration process proceeds.

The invention thus described attains the pre-established object and achieves important advantages.

The described device allows a process of regeneration of the skin that is quick and does not require drawing healthy skin from the patient.

Clearly, this allows use also in case of very severe burns, after which healthy skin is not available. Moreover, it has been found that the reconstructed skin zone has less scarring, therefore offering a more aesthetically pleasing result.

## Claims

1. Device (1) for skin reconstruction of patient who suffered burns **characterized in that** it comprises a piece of fabric (2) to be placed above a portion of a patient body, made of PGA, said piece of fabric (2) being perforated, for a simultaneous protection of the portion damaged by the burn and oxygenation of the tissues, with a plurality of holes (3) arranged over the entire surface of said piece of fabric (2), being all equivalent to each other for shape and size, equidistant from each other and arranged along straight lines so as to form a regular lattice, at least one of the surfaces of the piece (2) comprising at least one coating layer (4) being made of collagen.

2. Device according to claim 1, **characterized in that** said holes (3) have rhomboid shape.

3. Device according to any one of the preceding claims, **characterized in that** said holes (3) have one side with length comprised between 0.5 cm and 1 cm.

4. Device according to any one of the preceding claims, **characterized in that** the distance between said adjacent holes (3) is comprised between 0.5 cm and 1 cm.

5. Device according to any one of the preceding claims, **characterized in that** the fabric of said piece (2) is of warp knitted type and/or preferably textured.

6. Device according to any one of the preceding claims, **characterized in that** the thickness of the fabric of said piece (2) is comprised between 0.1 mm and 2 mm, preferably between 0.3 mm and 0.6 mm, more preferably between 0.4 mm and 0.53 mm, still more preferably is 0.45 mm.

7. Device according to any one of the preceding claims, **characterized in that** the fabric of said piece (2) is obtained with a thread having a density comprised between 50 and 200 denier.

## Patentansprüche

1. Vorrichtung (1) zur Wiederherstellung der Haut eines Patienten, der an Brandwunden gelitten hat, **dadurch gekennzeichnet, dass** sie ein Stoffstück (2) umfasst, das auf einen Abschnitt eines Patientenkörpers platziert werden muss und aus PGA (Polyglycolsäure) besteht, wobei das Stoffstück (2) perforiert ist, für einen gleichzeitigen Schutz des durch die Brandwunde beschädigten Abschnitts und für die Oxygenierung der Gewebe, mit einer Vielzahl von Löchern (3), die auf der gesamten Fläche des Stoffstücks (2) angeordnet sind, wobei sie alle einander äquivalent sind, was die Form und die Größe betrifft, in den gleichen Abständen voneinander sind und entlang gerader Linien angeordnet sind, um einen regelmäßigen Gitter zu bilden, wobei zumindest eine der Flächen des Stückes (2) zumindest eine Deckschicht (4) umfasst, die aus Collagen besteht.

2. Vorrichtung nach Anspruch 1, **dadurch gekennzeichnet, dass** die Löcher (3) eine rhomboide Form haben.

3. Vorrichtung nach einem der vorhergehenden Ansprüche, **dadurch gekennzeichnet, dass** die Löcher (3) eine Seite mit einer Länge haben, die zwischen 0,5 cm und 1 cm liegt.

4. Vorrichtung nach einem der vorhergehenden Ansprüche, **dadurch gekennzeichnet, dass** der Abstand zwischen den begrenzenden Löchern (3) zwischen 0,5 cm und 1 cm liegt.

5. Vorrichtung nach einem der vorhergehenden Ansprüche, **dadurch gekennzeichnet, dass** der Stoff des Stückes (2) vom Kettengewirkten Typ und/oder vorzugsweise texturiert ist.

6. Vorrichtung nach einem der vorhergehenden Ansprüche, **dadurch gekennzeichnet, dass** die Dicke des Stoffes des Stückes (2) zwischen 0,1 mm und 2 mm, bevorzugt zwischen 0,3 mm und 0,6 mm, weiter bevorzugt zwischen 0,4 mm und 0,53 mm liegt, noch weiter bevorzugt 0,45 mm ist.

7. Vorrichtung nach einem der vorhergehenden Ansprüche, **dadurch gekennzeichnet, dass** der Stoff des Stückes (2) mit einem Faden erhalten wird, der eine Dichte hat, die zwischen 50 und 200 Denier liegt.

## Revendications

1. Dispositif (1) pour la reconstruction d'un peau of patient qui a souffert de brûlures, **caractérisé en ce qu'**il comprend une pièce de tissu (2) à placer sur une partie du corps du patient, réalisé en PGA, ladite pièce de tissu (2) étant perforée, pour une protection simultanée de la partie endommagée suite à la brûlure et pour l'oxygénation des tissus, avec une pluralité de trous (3) disposés sur toute la surface de ladite pièce de tissu (2), qui sont tous équivalents entre eux en ce qui concerne la forme et la dimension, ils sont équidistants les uns des autres et ils sont disposés le long de lignes droites, de sorte à former un réseau régulier, au moins une des surfaces de la pièce (2) comprenant au moins une couche de revêtement (4) réalisée en collagène.

2. Dispositif selon la revendication 1, **caractérisé en ce que** lesdits trous (3) ont une forme de losange.

3. Dispositif selon l'une quelconque des revendications précédentes, **caractérisé en ce que** lesdits trous (3) ont un côté avec une longueur comprise entre 0,5 cm et 1 cm.

4. Dispositif selon l'une quelconque des revendications précédentes, **caractérisé en ce que** ladite distance entre lesdits trous adjacents (3) est comprise entre 0,5 cm et 1 cm.

5. Dispositif selon l'une quelconque des revendications précédentes, **caractérisé en ce que** le tissu de ladite pièce (2) est du type tricoté et/ou de préférence texturé.

6. Dispositif selon l'une quelconque des revendications précédentes, **caractérisé en ce que** l'épaisseur du tissu de ladite pièce (2) est comprise entre 0,1 mm et 2 mm, de préférence entre 0,3 mm et 0,6 mm, plus préférablement entre 0,4 mm et 0,53 mm, encore plus préférablement il est 0,45 mm.

7. Dispositif selon l'une quelconque des revendications précédentes, **caractérisé en ce que** le tissu de ladite pièce (2) est obtenu avec un fil ayant une densité comprise entre 50 et 200 deniers.
